# EUROPEAN PATENT SPECIFICATION

(11) **EP 4 498 919 B1**
(45) Date of publication and mention of the grant of the patent: **06.05.2026**
(21) Application number: 23712021.7
(22) Date of filing: 20.03.2023
(51) Int. Cl.: A61B 6/50, A61B 6/03, A61B 6/00

(54) **CONTRAST AGENT ATTENUATION GRADIENT**
KONTRASTMITTEL-ABSCHWÄCHUNGSGRADIENT
GRADIENT D'ATTÉNUATION D'AGENT DE CONTRASTE

(30) Priority: 31.03.2022 EP 22166012
(43) Date of publication of application: 05.02.2025
(73) Proprietor: Koninklijke Philips N.V., 5656 AG Eindhoven (NL)
(72) Inventor: SCHMITT, Holger, 5656 AG Eindhoven (NL); LANGZAM, Eran, 5656 AG Eindhoven (NL); GRASS, Michael, 5656 AG Eindhoven (NL); HAASE, Christian, 5656 AG Eindhoven (NL); NICKISCH, Hannes, 5656AG Eindhoven (NL)
(74) Representative: Philips Intellectual Property & Standards
(86) International application number: PCT/EP2023/056989
(87) International publication number: WO 2023/186589

(56) References cited:
- US-A1- 2014 088 414
- US-A1- 2014 243 662
- US-A1- 2020 237 329

## Description

### TECHNICAL FIELD

The present disclosure relates to calculating a value of a contrast agent attenuation gradient for a lumen in a vasculature. A computer-implemented method, a computer program product, and a system, are disclosed.

### BACKGROUND

Various clinical investigations involve performing an assessment of blood flow in the vasculature. For example, investigations for coronary artery diseases "CAD" often perform an assessment of blood flow. In this regard, various blood flow parameters have been investigated, including the Fractional Flow Reserve "FFR", the instantaneous wave-free ratio "iFR", the Coronary Flow Reserve "CFR", the Thrombolysis in Myocardial Infarction "TIMI" flow grade, the Index of Microvascular Resistance "IMR", and the Hyperemic Microvascular Resistance index "HMR".

Such blood flow parameters have historically been measured using invasive devices such as a pressure-wire. However, more recently, angiographic measurements have been used. By way of an example, the Fractional Flow Reserve "FFR" is often determined in order to assess the impact of a stenosis on delivery of oxygen to the heart muscle in a CAD assessment. The FFR is defined by the ratio *P_{d}*/*Pₐ*, wherein *P_{d}* represents a distal pressure at a distal position with respect to the stenosis, and *Pₐ* represents a proximal pressure with respect to the stenosis. Historically, values for these pressures have been determined by positioning an invasive device, such as a pressure wire, at the respective positions in the vasculature. However, more recently, angiographic techniques for determining the FFR have been developed. According to fluid flow theory, pressure changes are linked to changes in fluid velocity. In the example of the FFR, angiographic images of an injected contrast agent may be analyzed in order to determine the blood flow velocity. The FFR may then be calculated by using a haemodynamic model to estimate pressure values in the lumen from the blood flow velocity. Thus, the FFR, as well as other blood flow parameters may be determined angiographically.

A challenge with such angiographic techniques for assessing blood flow in a vasculature is the need to provide an accurate angiographic model that represents the lumen under investigation. An alternative angiographic technique, and which does not necessitate such a haemodynamic model, is based on a measurement of the attenuation gradient of an injected contrast agent, the attenuation gradient being measured along the lumen. In this regard, a document by Wong, D. T. L, et al., "Transluminal Attenuation Gradient in Coronary Computed Tomography Angiography Is a Novel Noninvasive Approach to the Identification of Functionally Significant Coronary Artery Stenosis: A Comparison With Fractional Flow Reserve", JACC Vol. 61, No. 12, 2013, pates 1271 - 1279, reports on a technique that has recently received increased clinical interest.

The technique disclosed in the aforementioned document uses a single static cardiac CT image as input. The coronary arteries are segmented in this three-dimensional image, and the gradient of the Hounsfield Unit contrast agent attenuation "transluminal attenuation gradient" is determined at multiple positions along the centerlines of the coronary arteries. The magnitude of the gradient has been found to correlate with stenoses, i.e. narrowings, in the arterial lumen. The document concludes that angiographic measurements of the attenuation gradient along a lumen provide an acceptable prediction of invasive FFR measurements, and may provide a non-invasive technique for detecting functionally significant coronary stenoses.

A document US 2014/0243662 A1 describes a method for non-invasively determining the functional severity of arterial stenosis in a selected portion of an arterial network. The method includes gathering patient-specific data related to concentration of a contrast agent within an arterial network using a coronary computed tomography angiography scan. The data can be gathered under rest or stress conditions. Estimation of a loss coefficient can be used to eliminate the need for data gathered under stress. The data is used to calculate a transluminal attenuation gradient. The data may be corrected for imaging artifacts at any stage of the analysis. Transluminal attenuation flow encoding is used to determine an estimate of flow velocity. Once velocity is determined, pressure gradient, coronary flow reserve, and/or fractional flow reserve can be determined through a variety of methods. These estimates can be used to estimate functional severity of stenosis.

However, there remains room to improve the accuracy of angiographic measurements of the contrast agent attenuation gradient in a lumen, and to thereby provide more accurate measurements of blood flow parameters for the lumen, such as for example the FFR.

### SUMMARY

According to one aspect of the present disclosure, a computer-implemented method of calculating a value of a contrast agent transluminal attenuation gradient for a lumen in a vasculature, is provided. The method includes:
receiving spectral computed tomography, CT, data representing a distribution of an injected contrast agent along the lumen, the spectral CT data defining X-ray attenuation at a plurality of energy intervals;
analyzing the spectral CT data to isolate from the spectral CT data, contrast agent attenuation data representing the distribution of the contrast agent along the lumen;
calculating, from the contrast agent attenuation data, an average value of the contrast agent attenuation across the lumen, at a plurality of positions along the lumen; and
calculating a value of a gradient of the contrast agent along one or more portions of the lumen using the average values of the contrast agent attenuation across the lumen, to provide the value of the contrast agent transluminal attenuation gradient.

Since, in the above method, the value of a gradient of the contrast agent along the lumen is calculated using contrast agent attenuation data that is isolated from spectral CT data, improved isolation is provided between attenuation arising from the contrast agent, and attenuation arising from background materials such as fat, water, soft tissue, calcified plaque, bone, and metals that may also be present in the vicinity of the vasculature. The isolation is improved in comparison to techniques that use the Hounsfield Unit values from conventional CT data to represent contrast agent attenuation. Consequently, the accuracy of the calculated gradient of the contrast agent, is improved. This, in turn, improves the accuracy of measurements of blood flow parameters for the lumen that are determined using the calculated value of the gradient.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of calculating a value of a contrast agent attenuation gradient for a lumen in a vasculature, in accordance with some aspects of the present disclosure.
Fig. 2 is a schematic diagram illustrating an example of a system 200 for calculating a value of a contrast agent attenuation gradient for a lumen in a vasculature, in accordance with some aspects of the present disclosure.
Fig. 3 is a schematic diagram illustrating a heart, including an example of a lumen 120, in accordance with some aspects of the present disclosure.
Fig. 4 is a schematic diagram illustrating an example of a spectral CT imaging system 220 including an X-ray detector 230, in accordance with some aspects of the present disclosure.
Fig. 5 is a graph illustrating the dependence of the mass attenuation coefficient with X-ray energy for two example materials, iodine and water.
Fig. 6 illustrates examples of images that are reconstructed from spectral CT data 130₁, 130₂ representing a distribution of an injected contrast agent along a lumen 120, and in which the spectral CT data defines X-ray attenuation A) at a first, relatively lower energy interval ΔE₁, and B) at a second, relatively higher energy interval ΔE₂, in accordance with some aspects of the present disclosure.
Fig. 7 illustrates an example of an image that is reconstructed from contrast agent attenuation data 140 representing a distribution of contrast agent along a lumen, and which has been isolated from the spectral CT data 130₁, 130₂ illustrated in Fig. 6, in accordance with some aspects of the present disclosure.
Fig. 8 illustrates an example of a graph representing a distribution of a contrast agent, CA, along a lumen 120, in accordance with some aspects of the present disclosure.
Fig. 9 illustrates an example of a neural network NN that is trained to predict a value of a gradient of a contrast agent along the lumen, in accordance with some aspects of the present disclosure.
Fig. 10 illustrates an example of a reconstructed image slice that is reconstructed from contrast agent attenuation flow data 160 representing a flow of an injected contrast agent in a lumen 120, in accordance with some aspects of the present disclosure.
Fig. 11 is an example of a planar image 180 including a lumen 120 and one or more anatomical landmarks, in accordance with some aspects of the present disclosure.
Fig. 12 is an example of a planar planning image 190 representing tissue data, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a method of calculating a value of a contrast agent attenuation gradient for a lumen in a vasculature. In some examples, the vasculature is the coronary vasculature, and the lumen is the lumen of a coronary vessel. In some examples, the vessel is a coronary artery. However, it is to be appreciated that the coronary vessel may alternatively be a coronary vein. More generally, the method may be used to calculate a value of a contrast agent attenuation gradient for a lumen in the vasculature in another part of the body than the heart. For example, the lumen may alternatively be a lumen of a vessel, i.e., a vein or an artery, that is located in the leg, the arm, the brain, and so forth.

Reference is also made herein to examples in which the calculated contrast agent attenuation gradient is used to determine a fractional flow reserve "FFR" value for the lumen. The FFR is an example of a blood flow parameter. It is, however, to be appreciated that the calculated contrast agent attenuation gradient may be used to determine a value of other types of blood flow parameters for the lumen. For instance, the calculated contrast agent attenuation gradient may alternatively be used to determine the value of blood flow parameters such as, and without limitation, an iFR value, a CFR value, a TIMI flow grade, an IMR value, and an HMR value, a volumetric blood flow value, a hyperemic stenosis resistance "HSR" value, a zero flow pressure "ZFP" value, and an instantaneous hyperemic diastolic velocity-pressure slope "IHDVPS" value.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, there remains room to improve the accuracy of angiographic measurements of the contrast agent attenuation gradient in a lumen, and to thereby provide more accurate measurements of blood flow parameters for the lumen, such as for example the FFR.

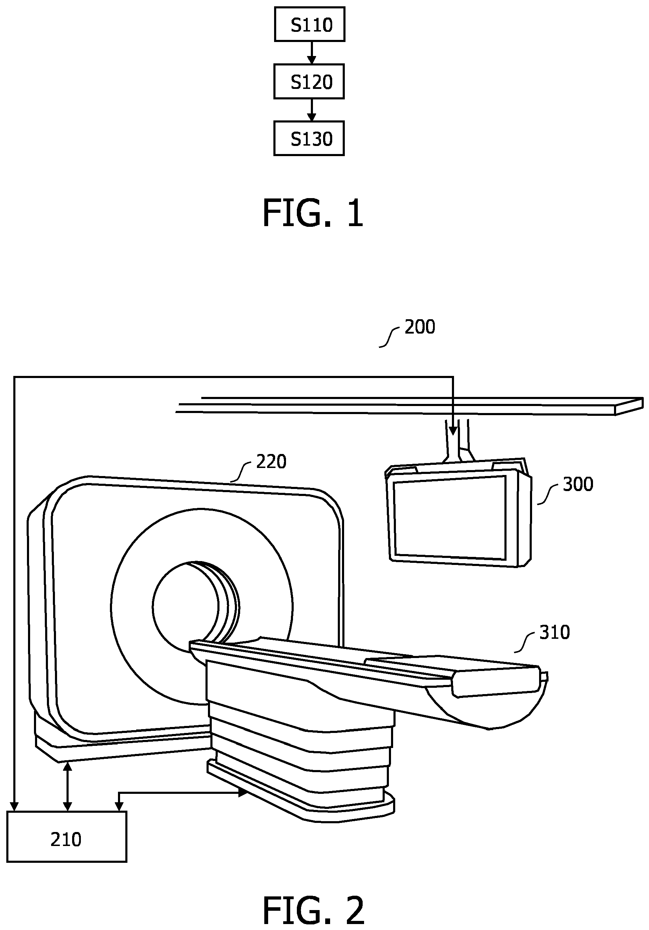

Fig. 1 is a flowchart illustrating an example of a computer-implemented method of calculating a value of a contrast agent attenuation gradient for a lumen in a vasculature, in accordance with some aspects of the present disclosure. Fig. 2 is a schematic diagram illustrating an example of a system 200 for calculating a value of a contrast agent attenuation gradient for a lumen in a vasculature, in accordance with some aspects of the present disclosure. Operations described in relation to the method illustrated in Fig. 1, may also be performed in the system 200 illustrated in Fig. 2, and vice versa. With reference to Fig. 1, the computer-implemented method of calculating a value of a contrast agent attenuation gradient 110 for a lumen 120 in a vasculature includes:
receiving S110 spectral computed tomography, CT, data 130₁, 130₂ representing a distribution of an injected contrast agent along the lumen 120, the spectral CT data defining X-ray attenuation at a plurality of energy intervals ΔE_{1..m};
analyzing S120 the spectral CT data to isolate from the spectral CT data, contrast agent attenuation data 140 representing the distribution of the contrast agent along the lumen 120; and
calculating S130, from the contrast agent attenuation data 140, a value of a gradient 110 of the contrast agent along one or more portions Pₐ - P_{d} of the lumen, to provide the value of the contrast agent attenuation gradient 110.

Since, in the above method, the value of a gradient of the contrast agent along the lumen is calculated using contrast agent attenuation data that is isolated from spectral CT data, improved isolation is provided between attenuation arising from the contrast agent, and attenuation arising from background materials such as fat, water, soft tissue, calcified plaque, bone, and metals that may also be present in the vicinity of the vasculature. The isolation is improved in comparison to techniques that use the Hounsfield Unit values from conventional CT data to represent contrast agent attenuation. Consequently, the accuracy of the calculated gradient of the contrast agent, is improved. This, in turn, improves the accuracy of measurements of blood flow parameters for the lumen that are determined using the calculated value of the gradient.

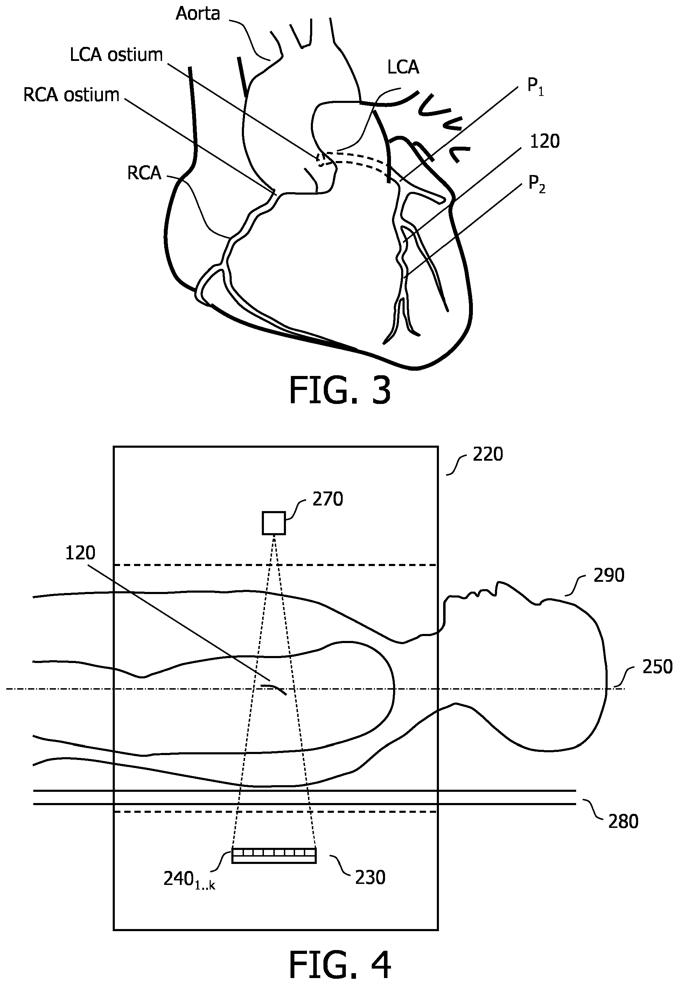

The above method is also described with reference to Fig. 3, which is a schematic diagram illustrating a heart, including an example of a lumen 120, in accordance with some aspects of the present disclosure. The heart illustrated in Fig. 3 is labelled with the left coronary artery, LCA, the right coronary artery, RCA, and the LCA ostium and RCA ostium that respectively define the openings of these arteries in the aorta. By way of an example, the operations described above with reference to Fig. 1 may be performed in order to calculate a value of a contrast agent attenuation gradient for the lumen 120 of the left coronary artery, LCA, illustrated in Fig. 3.

Returning to the method illustrated in Fig. 1, in the operation S110, spectral computed tomography, CT, data 130₁, 130₂, is received. The spectral CT data represents a distribution of an injected contrast agent along a lumen 120. The spectral CT data may for instance represent the distribution of an injected contrast agent along a portion of the lumen 120 illustrated in Fig. 3. By way of some examples, the spectral CT data may represent the distribution of an injected contrast agent along the portion of the lumen 120 between the two positions P₁ and P₂ in the lumen 120, or between the ostium of the left coronary artery "LCA ostium" and another position, such as the position P₁ or the position P₂.

The spectral CT data 130₁, 130₂ that is received in the operation S110 may be received by the one or more processors 210 illustrated in Fig. 2. In general, the one or more processors 210 may receive the spectral CT data 130₁, 130₂ from a spectral X-ray imaging system, or from another source, such as a computer readable storage medium, the Internet, or the Cloud, for example. The spectral CT data 130₁, 130₂ may be received from the spectral CT imaging system 220 illustrated in Fig. 2, for example. The spectral CT data 130₁, 130₂ may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

With reference to the method illustrated in Fig. 1, the spectral CT data 130₁, 130₂, that is received in the operation S110 represents a distribution of an injected contrast agent along a lumen 120. In this regard, the spectral CT data may be generated subsequent to the injection of a contrast agent into the vasculature. The contrast agent may include a substance such as iodine, or a Lanthanide such as gadolinium, or indeed another substance that provides visibility of a flow in the vasculature into which the contrast agent is injected.

With continued reference to the method illustrated in Fig. 1, the spectral CT data 130₁, 130₂, that is received in the operation S110 may in general be generated by a spectral X-ray imaging system, i.e. a spectral CT imaging system, or a spectral X-ray projection imaging system.

A spectral CT imaging system generates spectral CT data whilst rotating, or stepping, an X-ray source-detector arrangement around an imaging region. Examples of spectral CT imaging systems include cone beam spectral CT imaging systems, photon counting spectral CT imaging systems, dark-field spectral CT imaging systems, and phase contrast spectral CT imaging systems. By way of an example, the spectral CT data 130₁, 130₂, may be generated by the Spectral CT 7500 that is marketed by Philips Healthcare, Best, The Netherlands.

An example of a spectral CT imaging system 220 that may be used to generate the spectral CT data that is received in the operation S110, is illustrated in Fig. 2. Another example of such a spectral CT imaging system 220 is illustrated in Fig. 4. Fig. 4 is a schematic diagram illustrating an example of a spectral CT imaging system 220 including an X-ray detector 230, in accordance with some aspects of the present disclosure. The spectral CT imaging system 220 illustrated in Fig. 4 includes an X-ray source 270 and the X-ray detector 230. The X-ray source 270 and X-ray detector 230 are mechanically coupled to a gantry (not illustrated in Fig. 4). In operation, the X-ray source 270 and X-ray detector 230 are rotated by means of the gantry around an axis of rotation 250 whilst acquiring spectral CT data representing X-ray attenuation within a portion of an object, such as a subject 290. The spectral CT data obtained from multiple rotational angles around the axis of rotation may be subsequently reconstructed into a volumetric image. As illustrated in Fig. 4, the X-ray detector 230 may include a plurality of detector elements 240_{1..k} arranged along an axis of rotation 250 of the X-ray detector. The detector elements 240_{1..k} may for example be arranged parallel to, or at an acute angle with respect to the axis of rotation. This arrangement allows the X-ray detector 230 to capture spectral CT data that represents a flow of a contrast agent in a direction along the axis of rotation, such as may occur in some of the vessels in the heart, for instance.

As mentioned above, the spectral CT data that is received in the operation S110 may alternatively be generated by a spectral X-ray projection imaging system. Spectral X-ray projection imaging systems typically include a support arm such as a so-called "C-arm" that supports an X-ray source and an X-ray detector. Spectral X-ray projection imaging systems may alternatively include a support arm with a different shape to this example, such as an O-arm, for example. Spectral X-ray projection imaging systems typically generate spectral CT data with the support arm held in a static position with respect to an imaging region during the acquisition of image data. However, spectral X-ray projection imaging systems may also acquire spectral CT data whilst rotating their support arm around an axis of rotation. Image reconstruction techniques may then be used to reconstruct this data into a volumetric image in a similar manner to a spectral CT imaging system. Thus the spectral CT data that is received in the operation S110 may alternatively be generated by a spectral X-ray projection imaging system.

The spectral CT data 130₁, 130₂, that is received in the operation S110 defines X-ray attenuation at a plurality of energy intervals ΔE_{1..m}. In general there may be two or more different energy intervals; i.e. *m* is an integer, and *m* ≥ 2.The ability to generate X-ray attenuation data at multiple different energy intervals ΔE_{1..m} distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable in conventional X-ray image data. In this regard, various different configurations of spectral X-ray imaging systems may be used to generate the spectral CT data that is received in the operation S110, some of which are described with reference to Fig. 4.

With reference to Fig. 4, in general, the X-ray source 270 may include multiple monochromatic sources, or one or more polychromatic sources, and the X-ray detector 230 may include: a common detector for detecting multiple different X-ray energy intervals, or multiple detectors wherein each detector detects a different X-ray energy interval ΔE_{1..m}, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources 270 and detectors 230 may be used to detect X-rays within different X-ray energy intervals ΔE_{1..m}. In general, discrimination between different X-ray energy intervals may be provided at the source 270 by temporally switching the X-ray tube potential of a single X-ray source 270, i.e. "rapid kVp switching", or by temporally switching, or filtering, the emission of X-rays from multiple X-ray sources. In such configurations, a common X-ray detector may be used to detect X-rays across multiple different energy intervals, attenuation data for each energy interval being generated in a time-sequential manner. Alternatively, discrimination between different X-ray energy intervals may be provided at the detector 230 by using a multi-layer detector, or a photon counting detector. Such detectors can detect X-rays from multiple X-ray energy intervals ΔE_{1..m} near-simultaneously, and thus there is no need to perform temporal switching at the source 270. Thus, a multi-layer detector, or a photon counting detector, may be used in combination with a polychromatic source to generate X-ray attenuation data at different X-ray energy intervals ΔE_{1..m}.

Other combinations of the aforementioned X-ray sources and detectors may also be used to provide the spectral CT data for the plurality of energy intervals ΔE_{1..m}. For example, in a yet further configuration, the need to sequentially switch different X-ray sources emitting X-rays at different energy intervals may be obviated by mounting X-ray source-detector pairs to a gantry at rotationally-offset positions around an axis of rotation. In this configuration, each source-detector pair operates independently, and separation between the spectral CT data for the different energy intervals ΔE_{1..m} is facilitated by virtue of the rotational offsets of the source-detector pairs. Improved separation between the spectral CT data for the different energy intervals ΔE_{1..m} may be achieved with this configuration by applying an energy-selective filter to the X-ray detector(s) in order to reduce the effects of X-ray scatter.

Returning to the method illustrated in Fig. 1, in the operation S 120, the received spectral CT data is then analyzed in order to isolate from the spectral CT data, contrast agent attenuation data 140 representing the distribution of the contrast agent along the lumen 120. This operation involves identifying as contrast agent attenuation data 140, portions of the spectral CT data that correspond to a material of the contrast agent, based on an energy-dependent X-ray attenuation signature of the material and/or based on an energy-dependent X-ray attenuation signature of one or more background materials represented in the spectral CT data.

The use of various "material decomposition" techniques is contemplated for use in analyzing the spectral CT data in the operation S120. The spectral CT data represents a distribution of the injected contrast agent along the lumen 120. The injected contrast agent may include a material such as iodine, or gadolinium. Such materials are often used as contrast agents in view of their attenuation at the X-ray energies used in diagnostic X-ray imaging systems. In addition to the attenuation arising from the contrast agent, the spectral CT data may also represent attenuation arising from one or more background materials such as fat, water, bone, soft tissue, vessel calcification, air, and metals such as gold, titanium, tungsten, and platinum. Such materials are often also present in the vicinity of the vasculature, and therefore attenuation arising from these materials may also be represented in the spectral CT data. For example, when imaging the cardiac vasculature, bone, in the form of portions of the spine, or ribs are often within the field of view of a spectral CT imaging system. Likewise, fiducial markers, implanted medical devices, and interventional devices are typically formed from metals such as those cited above, and attenuation arising from these materials may also be captured in the spectral CT data. By isolating the contrast agent attenuation data from the spectral CT data, more reliable data on the distribution of the injected contrast agent along the lumen 120 is obtained.

One example of a material decomposition technique that may be used in the operation S120 in order to isolate the contrast agent attenuation data 140 from the spectral CT data 130₁, 130₂, is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another suitable material decomposition technique is disclosed in a document by Roessl, E. and Proksa, R., "K-edge imaging in X-ray computed tomography using multi-bin photon counting detectors", Phys Med Biol. 2007 Aug 7, 52(15):4679-96. Another suitable material decomposition technique is disclosed in the published PCT patent application WO/2007/034359 A2. Another suitable material decomposition technique is disclosed in a document by Silva, A. C., et al., "Dual-energy (spectral) CT: applications in abdominal imaging", RadioGraphics 2011; 31(4):1031-1046.

In general, the X-ray attenuation spectrum of a material includes a contribution from Compton Scatter and a contribution from the Photo-electric effect. The attenuation due to Compton scatter is relatively similar for different materials, whereas the attenuation from the Photo-electric effect is strongly material-dependent. Both Compton Scatter and the Photo-electric effect exhibit an energy dependence, and it is this effect that is exploited by material decomposition techniques to analyze the spectral CT data in order to distinguish between different materials.

In general, material decomposition algorithms operate by decomposing the attenuation spectrum of an absorbing medium into contributions from an assumed set of "basis" materials. The energy-dependent X-ray attenuation of the assumed basis materials is typically modelled as a combination of absorption from Compton Scatter, and the Photo-electric effect. Some materials also have a k-absorption edge "k-edge" energy that is within the energy range used by diagnostic X-ray imaging systems, and this effect may also be exploited in order to distinguish between different materials. A spectral decomposition algorithm seeks to estimate an amount of each of the basis materials that is required to produce the measured amount of X-ray attenuation at the two or more energy intervals of the spectral CT data. Water and iodine are examples of basis materials that are often separated in clinical practice using a so-called two-material decomposition algorithm. Non-fat soft tissue, fat, and iodine, are examples of basis materials that are often separated in clinical practice using a three-material decomposition algorithm.

As mentioned above, if the k-absorption edge "k-edge" energy of any of the basis materials is within the energy range used by diagnostic X-ray imaging systems, i.e., approximately 30 - 120 keV, this can help to identify the contribution of a basis material. The k-edge energy for a material is defined as the minimum energy required for the Photo-electric event to occur with a k-shell electron. The k-edge occurs at a characteristic energy for each material. The k-edge energy for a material is marked by a sharp increase in its X-ray attenuation spectrum at X-ray energies corresponding to the k-edge energy value. The k-edge energies of many materials that are present in the human body are too low to be detected in a diagnostic X-ray imaging system. For example, the k-edge energies of hydrogen, carbon, oxygen, and nitrogen are at energies that are less than 1 keV. However, materials such as iodine (k-edge = 33.2 keV), gadolinium (50.2 keV), gold (80.7 keV), platinum (78.4 keV), tantalum (67.4 keV), holmium (55.6 keV), and molybdenum (k-edge = 20.0 keV) have k-edge energy values that permit their distinction in spectral CT data acquired from diagnostic X-ray imaging systems.

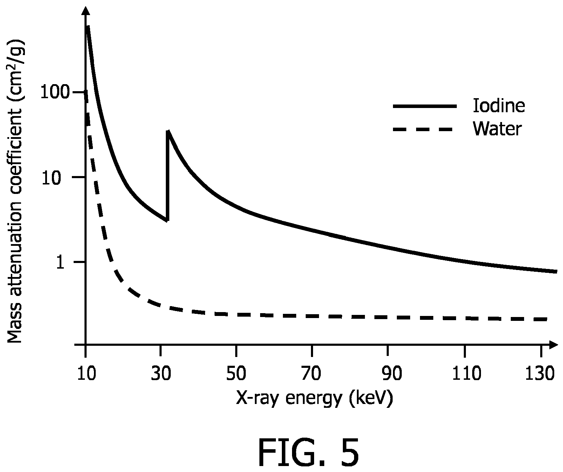

As mentioned above, materials such as iodine and gadolinium may be present in the vasculature as an injected contrast agent, and materials such as gold, platinum, tantalum, holmium, and molybdenum may be present in the vicinity of the vasculature body as fiducial markers, implanted medical devices, and interventional devices. Thus, the k-edge energies of such materials may be used to identify their presence in the spectral CT data 130₁, 130₂. By way of an example, Fig. 5 is a graph illustrating the dependence of the mass attenuation coefficient with X-ray energy for two example materials, iodine and water. The mass attenuation coefficient represented in Fig. 5 represents the X-ray attenuation. The sharp increase in X-ray attenuation for iodine at its k-edge energy of 33.2 keV facilitates a separation between the contributions of each of these materials to a combined attenuation spectrum. In this example, iodine and water may be separated by locating one energy interval ΔE₁ close to the k-edge energy of 33.2 keV, and another energy interval ΔE₂ significantly above the k-edge energy.

Thus, by using material decomposition techniques such as those described above, the contrast agent attenuation data 140 representing the distribution of the contrast agent along the lumen 120, may be isolated from the spectral CT data 130₁, 130₂.

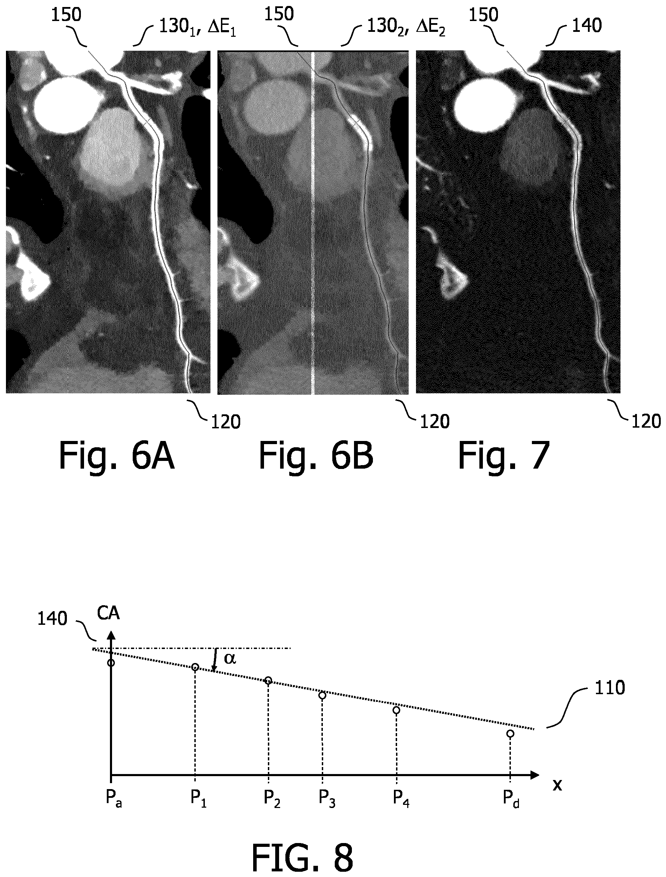

An example of the isolation of contrast agent attenuation data that is performed in the operation S120 is now described with reference to Fig. 6 and Fig. 7. In this example, the spectral CT data defines X-ray attenuation at two energy intervals, ΔE₁, and ΔE₂. Such spectral CT data is often referred-to as "dual energy" CT data. Fig. 6 illustrates examples of images that are reconstructed from spectral CT data 130₁, 130₂ representing a distribution of an injected contrast agent along a lumen 120, and in which the spectral CT data defines X-ray attenuation A) at a first, relatively lower energy interval ΔE₁, and B) at a second, relatively higher energy interval ΔE₂, in accordance with some aspects of the present disclosure. In Fig. 6, the spectral CT data for each of the energy intervals ΔE₁, and ΔE₂, has been reconstructed into the images illustrated in Fig. 6A and Fig, 6B respectively. The lumen 120 represented in these images is a coronary artery, and the lumen extends from the ostium, which is located in the light-shaded region at the top of the image, to the bottom of the image. In the illustrated example, the effects of attenuation arising from an iodine contrast agent, and a stent in the lumen are represented in the spectral CT data at the relatively lower energy interval ΔE₁, i.e. in the image in Fig. 6A. The attenuation arising from the stent in this image is masked by the attenuation arising from the contrast agent in the lumen. This relatively lower energy interval ΔE₁ may be used to identify the centerline of the lumen 120 since the iodine contrast agent is clearly visible. The effects of attenuation arising from iodine contrast agent tends to fade-out in the spectral CT data at the relatively higher energy interval ΔE₂, i.e. in the image in Fig. 6B. The relatively higher energy interval ΔE₂ can be used to identify objects with relatively higher density than tissue, such as the stent that appears in white in the upper portion of the lumen 120 illustrated in Fig. 6B. The data corresponding to the relatively higher energy interval ΔE₂ may be considered to represent attenuation arising from artifacts such as stents, calcifications, and metal objects such as (guide)wires, and implants, and which have a relatively higher density than the tissue surrounding the lumen 120.

Fig. 7 illustrates an example of an image that is reconstructed from contrast agent attenuation data 140 representing a distribution of contrast agent along a lumen, and which has been isolated from the spectral CT data 130₁, 130₂ illustrated in Fig. 6, in accordance with some aspects of the present disclosure. In the illustrated example, the contrast agent attenuation data 140 illustrated in Fig. 7 is isolated from the spectral CT data 130₁, 130₂ illustrated in Fig. 6, by subtracting the data corresponding to the relatively higher energy interval ΔE₂ from the data corresponding to the relatively lower energy interval ΔE₁. The pixel intensities in this image give a more accurate representation of X-ray attenuation arising from the contrast agent in the lumen because the effects of attenuation from confounding factors such as the stent (illustrated in Fig. 6B), calcifications, and (guide)wires has been reduced. This in turn leads to a more accurate calculation of the gradient of the contrast agent along the lumen in the operation S130.

In the example described above, the contrast agent attenuation data was isolated from spectral CT data that included only two energy intervals ΔE_{1..m}. In general, the use of more than two spectral CT data energy intervals ΔE_{1..m} may improve the isolation of the contrast agent attenuation data from the spectral CT data. Material decomposition techniques such as those described above may use the additional energy intervals to e.g. generate z-effective images that are specific to particular materials, and the image intensities of these images may be used to reduce the contribution to the contrast agent attenuation data from materials other than the contrast agent material. However, the effect of attenuation arising from some artifacts may still remain in the contrast agent attenuation data. For instance, the contrast agent attenuation data may include a contribution from artifacts such as stents, calcifications, and metal objects such as (guide)wires, and implants. A further reduction in the effects of these artifacts is also desirable in order to further improve the accuracy of the gradient of the contrast agent along the lumen in the operation S130. In one example, the isolated contrast agent attenuation data is further weighted using artifact attenuation data in order to reduce an impact of the artifacts on the isolated contrast agent attenuation data 140. In this example, the operation of analyzing S120 the spectral CT data, further includes:
analyzing the spectral CT data 130₁, 130₂ to isolate from the spectral CT data, artifact attenuation data representing attenuation arising from one or more artifacts; and
weighting the isolated contrast agent attenuation data such that an impact of the artifacts on the isolated contrast agent attenuation data 140 is reduced.

The weighting may for instance be applied based on a distance between the one or more artifacts and the centerline of the lumen. With reference to the example above and in which there are two energy intervals, ΔE₁ and ΔE₂; in this example, the artifact attenuation data may be provided by the spectral CT data corresponding to the relatively higher energy interval ΔE₂. Contributions to the data at the relatively higher energy interval ΔE₂ that arise from positions that are close to the centerline of the lumen may be validly assumed to represent attenuation arising from contrast agent since only contrast agent is expected to be present in the lumen at this position. By contrast, contributions to this data that arise from positions that are relatively further from the centerline of the lumen, may be assumed to be true artifacts. Therefore, when weighting the isolated contrast agent attenuation data in order to reduce an impact of the artifacts on the isolated contrast agent attenuation data 140, a relatively higher weighting may be applied to artifacts in positions that are relatively further from the centerline of the lumen than to artifacts in positions that are relatively close to the centerline of the lumen. In so doing, the isolated contrast agent attenuation data may more accurately represent attenuation arising from the contrast agent. Thus, in a related example, the one or more spectral CT data energy intervals ΔE_{1..m} comprises a relatively lower energy interval ΔE₁ and a relatively higher energy interval ΔE₂; and
the contrast agent attenuation data 140 is isolated from the spectral CT data 130₁, 130₂ by subtracting the spectral CT data corresponding to the relatively higher energy interval ΔE₂ from the spectral CT data corresponding to the relatively lower energy interval ΔE₁; and
the artifact attenuation data 140 is provided by the spectral CT data corresponding to the relatively higher energy interval ΔE₂.

Returning to the method illustrated in Fig. 1, in the operation S130, a value of a gradient 110 of the contrast agent along one or more portions of the lumen, is then calculated from the contrast agent attenuation data 140, to provide the value of the contrast agent attenuation gradient 110. In this operation, the value of the gradient 110 is calculated in a direction that extends along the lumen. The calculated value(s) of the gradient may then be outputted. The calculated value(s) of the gradient may be outputted to a computer readable storage medium, or to a printer, or to a display device, such as to the monitor 300 illustrated in Fig. 2, for example.

An example of the operation S130 is described with reference to Fig. 8, which illustrates an example of a graph representing a distribution of a contrast agent, CA, along a lumen 120, in accordance with some aspects of the present disclosure. The graph illustrated in Fig. 8 represents the X-ray attenuation arising from the contrast agent, CA, on the vertical-axis, as a function of position along the lumen, x, on the horizontal axis. Various example measurements of the X-ray attenuation arising from the contrast agent are indicated at the positions, Pₐ, P_{1..4}, and P_{d}. The position Pₐ, P_{1..4}, and P_{d} represent positions along a lumen, such as the lumen 120 illustrated in Fig. 7. The graph illustrated in Fig. 8 may be generated by sampling, at the positions Pₐ, P_{1..4}, and P_{d}, the contrast agent attenuation data that corresponds to the reconstructed image illustrated in Fig. 7. The position Pₐ may for instance represent a position in the ostium of the lumen. The position P₁ may represent a position that is proximal to a stenosis at position P₂, the positions P₃ and P₄ may represent positions that are distal to the stenosis. The position P_{d} may represent a position in of the lumen that is even further downstream of the stenosis. In the example illustrated in Fig. 8, the X-ray attenuation arising from the contrast agent, CA, decreases between the position Pₐ and the position P_{d}. This is due to the presence of the stenosis at the position P₂. A gradient 110 may be calculated for the portion of the vessel, such as the portion between the positions Pₐ and P_{d}, i.e. for the portion Pₐ - P_{d}. The gradient 110 may be calculated by dividing the difference in X-ray attenuation between the positions Pₐ and P_{d}, by the length of the lumen between these positions. The value of this gradient has been found to correlate with the FFR value for the lumen, as reported in the document by Wong, D. T. L., et al., cited above, and which reported this correlation for conventional CT data.

It is noted that the value of the gradient of the contrast agent may alternatively be calculated along other portions of the lumen than the example portion Pₐ - P_{d} described above. For example, the value of the gradient of the contrast agent may alternatively be calculated along the portion of the lumen Pₐ - P₁, i.e. between the positions Pₐ and P₁, or along the portion of the lumen P₂ - P₃, i.e. between the positions P₂ and P₃, or along another portion. A value of the gradient may also be calculated for multiple portions of the lumen. For instance, the values of the gradient may be calculated for multiple portions of the lumen such as the portions Pₐ - P₁, and P₁ - P₂, P₂ - P₃, P₃ - P₄, and P₄ - P_{d}. The value of the gradient may be calculated for multiple portions of the lumen so as to provide the gradient as a continuous function along a length of the lumen. By providing the values of the gradient along multiple portions of the lumen, for example as a continuous function, it may be possible to identify a position of the stenosis, or to provide further information for diagnosing a state of the lumen.

In general, the magnitude of the gradient 110 increases with the severity of the stenosis. Thus, in the absence of a stenosis, i.e. in a healthy vessel, similar values for the X-ray attenuation arising from the contrast agent, CA, may be expected along the vessel. In this situation, the X-ray attenuation arising from the contrast agent at the positions Pₐ, P_{1..4} and at P_{d} may be expected to be similar. In this situation, the gradient a, illustrated in Fig. 8 that is calculated along the portion of the vessel between the positions Pₐ and P_{d}, i.e. the portion Pₐ - P_{d}, may be expected to be approximately zero.

The value of the gradient may be calculated from the contrast agent attenuation data in various ways in the operation S130. In one example, the value of the gradient is calculated using a single value of the contrast agent at each of multiple positions Pₐ, P_{1..4} and at P_{d} along the lumen. The single value at each position may be determined on a centerline of the lumen, or alternatively it may be determined at an offset position with respect to the centerline. In another example, the value of the gradient is calculated using average values of the contrast agent attenuation across the lumen. In this example, the X-ray attenuation arising from the contrast agent, CA, plotted in Fig. 8, may represent the average value of the contrast agent attenuation across the lumen at each position, Pₐ, P₁₋₄, and P_{d}, along the lumen illustrated in Fig. 7. In this example, the attenuation gradient may be referred-to as a transluminal attenuation gradient; and the method described with reference to Fig. 1 further comprises:
calculating, from the contrast agent attenuation data 140, an average value of the contrast agent attenuation across the lumen 120, at a plurality of positions Pₐ, P₁₋₄, and P_{d}, along the lumen; and
wherein the calculating S130 a value of a gradient 110 of the contrast agent along one or more portions Pₐ - P_{d} of the lumen 120, is performed using the average values of the contrast agent attenuation across the lumen.

In a related example, a centerline of the lumen is also determined, and the average value of the contrast agent attenuation across the lumen is calculated at a plurality of positions along the centerline of the lumen. In this example, the X-ray attenuation arising from the contrast agent, CA, plotted in Fig. 8, represents the average value of the contrast agent attenuation across the lumen along the centerline, at each of the positions Pₐ, P₁₋₄, and P_{d}. In this example, the method further includes:
identifying a centerline 150 of the lumen 120; and wherein the calculating S130 an average value of the contrast agent attenuation across the lumen 120, at a plurality of positions Pₐ, P₁₋₄, and P_{d} along the lumen; is performed at the plurality of positions Pₐ, P₁₋₄, and P_{d} along the centerline of the lumen.
In this example, the centerline may be identified from the contrast agent attenuation data by determining the mid-point between the bilateral borders of the lumen. The centerline may alternatively be identified from the contrast agent attenuation data using a path-finding algorithm such as Fast Marching. A dynamic programming technique may be used to optimize the position of the centerline with respect to the borders of the lumen.

In another example, one or more positions across the lumen are identified. In this example, the method described with reference to Fig. 1 includes:
identifying one or more positions across the lumen 120; and
wherein the calculating S130 a value of a gradient 110 of the contrast agent along one or more portions Pₐ - P_{d} of the lumen 120, is performed at the one or more positions across the lumen, to provide the value of the contrast agent attenuation gradient 110.

In this example, a position across the lumen may for instance be a position on the centerline of the lumen. The position may alternatively be offset from the centerline. The position(s) may be defined with respect to the centerline by means of a grid, for example. Providing the value of the gradient at an offset position, or at multiple positions across the lumen, may facilitate a deeper understanding of the flow of the contrast agent in the lumen. In this example, the calculated value of the gradient may be outputted as an image representing the lumen and in which one or more "streamlines" indicate the magnitude of the gradient at each position, or as a colourmap in which the color corresponds to the value of the gradient, for example.

It is noted that the operation of calculating S130 a value of a gradient 110 of the contrast agent along one or more portions Pₐ - P_{d} of the lumen may in general be performed in the projection domain, or in the image domain. Thus, in the method described with reference to Fig. 1, the contrast agent attenuation data 140 may include projection data, i.e. raw data, or it may include reconstructed image data; and the calculating S130 a value of a gradient 110 of the contrast agent along one or more portions Pₐ - P_{d} of the lumen, may be performed in the projection domain, or in the image domain, respectively. By performing these operations in the projection domain, inaccuracies introduced during the image reconstruction process may be obviated, thereby resulting in a more accurate determination of the value of the gradient 110 of the contrast agent along the lumen 120. The projection data may also be processed at higher temporal resolution than reconstructed image data.

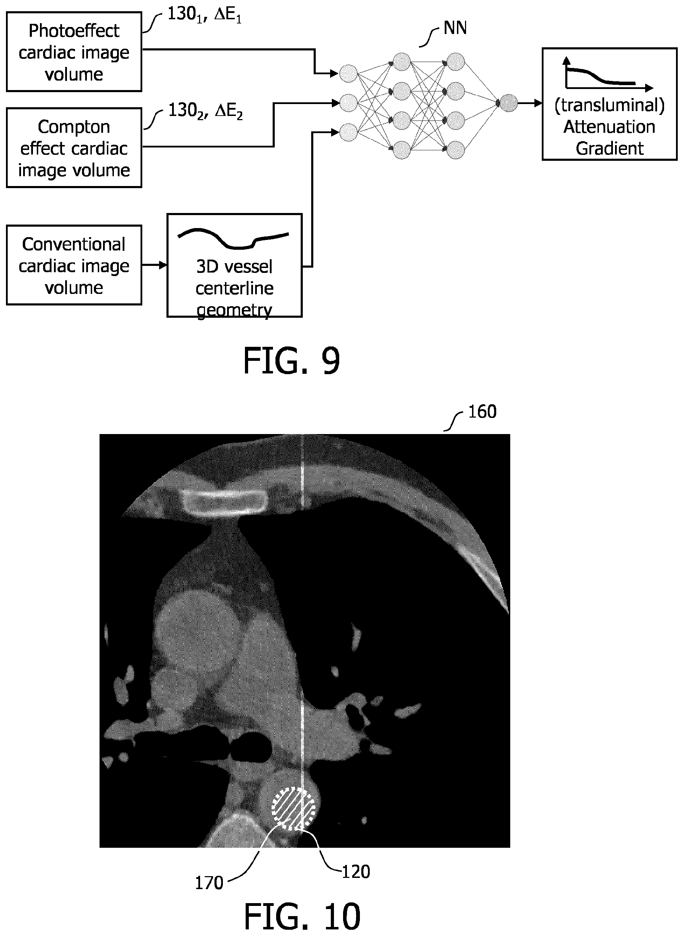

It is also noted that some of the elements, or even all of the elements of the analyzing operation S120 and/or the calculating operation S 130, may be performed by a neural network. Fig. 9 illustrates an example of a neural network NN that is trained to predict a value of a gradient of a contrast agent along the lumen, in accordance with some aspects of the present disclosure. With reference to the upper left portion of Fig. 9, spectral CT data at energy intervals ΔE₁ and ΔE₂ is inputted into the neural network NN. The spectral CT data may be inputted in the form of a reconstructed image, or as projection data. The reconstructed image may for instance correspond to the images illustrated in Fig. 6A and Fig. 6B, respectively. In the lower left portion of Fig. 9, a conventional cardiac volumetric image, is segmented, and the centerline of a lumen is extracted. The centerline may be extracted using the techniques described above, i.e. by determining the mid-point between the bilateral borders of the lumen, or by using a path finding algorithm such as Fast Marching. Data representing the lumen centerline, for example a volumetric image representing the centerline, is then inputted into the neural network NN. The operations of extracting a centerline of the lumen from image data may alternatively be performed using a neural network. The centerline may alternatively be extracted from data other than a conventional cardiac volumetric image. For instance, the centerline may be extracted from spectral CT data representing X-ray attenuation at one or more energy intervals. In this case, the spectral CT data representing X-ray attenuation at one or more of the energy intervals may be reconstructed into an image, segmented, and the centerline extracted from this reconstructed image.

The neural network NN illustrated in Fig. 9 may be provided by one or more architectures, such as a convolutional neural network "CNN", for example. The neural network NN may be trained to predict the (transluminal) attenuation gradient for the lumen by training the neural network NN using training image data in which the ground truth (transluminal) attenuation gradient has been determined by analyzing the training images. The training data, and consequently the ground truth data, may represent the lumens from multiple patients. The lumens from some tens, hundreds, or even thousands of patients may be used to train the neural network NN. The training data, and the ground truth data may represent patients having different ages, gender, body mass index, and so forth.

The inventors have also observed that the accuracy of contrast agent attenuation gradient measurements may be further improved by triggering the generation of the spectral CT data 130₁, 130₂, i.e. the data that is analyzed to provide the contrast agent attenuation data 140, using spectral CT data representing the flow of the injected contrast agent in the lumen.

In the technique for determining a transluminal attenuation gradient that is described in the document by Wong, D. T. L., et al., cited above, the generation of a static CT image is triggered based on the image intensity of a contrast agent bolus in a conventional CT image. The static CT image is generated when the image intensity in the conventional CT image reaches a predetermined Hounsfield Unit threshold value.

The inventors have observed that the time at which the spectral CT data 130₁, 130₂ is generated is important to providing accurate measurements of the contrast agent attenuation gradient. If the spectral CT data 130₁, 130₂ is generated too early, the gradient may not be detectable. If the spectral CT data 130₁, 130₂ is generated too late, the gradient may no longer be detectable. Moreover, it is important to trigger the generation of the spectral CT data 130₁, 130₂ at the same level of contrast agent across different patients in order to provide results that can be used in clinical studies. The inventors have determined that by triggering the generation of the spectral CT data 130₁, 130₂, i.e. the data that is analyzed to provide the contrast agent attenuation data 140, using spectral CT data representing the flow of the injected contrast agent in the lumen, rather than e.g. conventional CT data, the triggering accuracy can be improved because the impact of effects such as moving calcifications, and beam hardening from other objects on the X-ray attenuation data that is used to trigger the spectral CT data, is reduced.

In one example, the method described above with reference to Fig. 1 also includes:
receiving spectral CT data representing a flow of the injected contrast agent in the lumen 120;
analyzing the spectral CT data representing the flow of the injected contrast agent in the lumen to isolate from said data, contrast agent attenuation flow data 160 representing the flow of the injected contrast agent in the lumen 120; and
wherein the method further comprises:
   triggering a generation of the spectral CT data 130₁, 130₂ representing the distribution of the injected contrast agent along the lumen 120, if the injected contrast agent in the lumen represented in the contrast agent attenuation flow data satisfies a predetermined threshold condition.

In these operations, the receiving of the spectral CT data, and the analyzing of the spectral CT data to isolate the contrast agent attenuation flow data 160 may be performed in the same manner as described above in the operations S110 and S120. The spectral CT data representing a flow of the injected contrast agent in the lumen 120 may be generated by the same spectral X-ray imaging system as the spectral CT data 130₁, 130₂ representing the distribution of the injected contrast agent along the lumen 120. Similar material decomposition techniques may also be used to isolate the representing a flow of the injected contrast agent in the lumen 120 as described above in relation to the operation S130.

It is noted that this analyzing operation may in general be performed in the projection domain, or in the image domain. Thus, the contrast agent attenuation flow data 160 representing the flow of the injected contrast agent in the lumen 120 may include projection data, i.e. raw data, or it may include reconstructed image data. Consequently, the operation of triggering a generation of the spectral CT data 130₁, 130₂; may be performed in the projection domain, or in the image domain, respectively. By performing these operations in the projection domain, inaccuracies introduced during the image reconstruction process may be obviated, thereby resulting in a more accurate triggering of the generation of the spectral CT data 130₁, 130₂.

In this example, the generation of the spectral CT data 130₁, 130₂ may for instance be initiated if an intensity of the injected contrast agent represented in the contrast agent attenuation flow data 160 exceeds a predetermined threshold value at a predetermined position in the lumen 120. The position in the lumen may be the ostium of the lumen, for example. The value of the predetermined threshold may be set to a level such that the injected contrast agent would be expected to fill a lumen of interest. The predetermined threshold may represent a concentration of the contrast agent in the lumen. For example, the predetermined threshold may be set to trigger the generation of the spectral CT data 130₁, 130₂ at an iodine concentration of 15 milligrams per milliliter. As mentioned above, setting the predetermined threshold to a contrast agent concentration, rather than to a Hounsfield Unit attenuation value that is determined from conventional CT data, permits the generation of a gradient of the contrast agent in a repeatable manner, and consequently the gradient may be used in clinical studies. Thus in one example, the operation of triggering a generation of the spectral CT data 130₁, 130₂ representing the distribution of the injected contrast agent along the lumen 120, comprises initiating the generation of the spectral CT data 130₁, 130₂ representing the distribution of the injected contrast agent along the lumen 120, if an intensity of the injected contrast agent represented in the contrast agent attenuation flow data 160 exceeds a predetermined threshold value at a predetermined position in the lumen 120.

In some examples, the triggering of the generation of the spectral CT data 130₁, 130₂ is performed in the image domain. The triggering of the generation of the spectral CT data 130₁, 130₂ may be performed by monitoring the image intensity in a region of interest in a reconstructed image. In one example, an image slice is reconstructed, and the intensity of the injected contrast agent is monitored in a region of interest in the reconstructed image slice. Monitoring the image intensity in a reconstructed image slice has the advantage of reduced X-ray dose to a subject as compared to monitoring the image intensity in a reconstructed volumetric image. Thus, in one example, the received spectral CT data representing the flow of the injected contrast agent in the lumen 120 comprises projection data that is generated during a rotation of an X-ray source detector arrangement 270, 230 around the lumen 120. In this example, the method further comprises:
reconstructing the projection data into an image slice; and the initiating the generation of the spectral CT data 130₁, 130₂, comprises analyzing an image intensity within a region of interest 170 in the reconstructed image slice, and initiating the generation of the spectral CT data 130₁, 130₂ representing the distribution of the injected contrast agent along the lumen 120, if the image intensity within the region of interest exceeds the predetermined threshold value.

This example is described with reference to Fig. 10, which illustrates an example of a reconstructed image slice that is reconstructed from contrast agent attenuation flow data 160 representing a flow of an injected contrast agent in a lumen 120, in accordance with some aspects of the present disclosure. In the image illustrated in Fig. 10, the image slice is a slice through the descending aorta at mid-chest level. The lumen 120 is highlighted with the dotted circle, and the region of interest in this example is the area within the lumen 120. In this example, the image slice illustrated in Fig. 10 is reconstructed in real-time and continually updated. When the injected contrast agent reaches the region of interest 120, the intensity will change and the image intensity in the region of interest will change. A trigger signal may be generated based on e.g. the average, or the maximum intensity in the region of interest, and when this intensity exceeds the predetermine threshold level, the generation of the spectral CT data 130₁, 130₂ representing the distribution of the injected contrast agent along the lumen 120 is triggered. In so doing, the triggering of the spectral CT data occurs in a more consistent manner, and requires a lower X-ray dose. This is because a trigger signal is generated using contrast agent attenuation flow data 160 that is isolated from spectral CT data, i.e. with reduced interference from confounding effects of attenuation from calcifications and beam hardening, and because an image slice is used to generate the trigger signal rather than a volumetric image.

In this example, the region of interest 170 may be defined manually, or automatically. A manual identification of the region of interest 170 may be performed by means of a user operating a user input device in combination with the displayed reconstructed image slice. An automatic identification of the region of interest 170 may be performed using a feature detector, or a trained neural network.

In this example, a temporal profile of the intensity of the injected contrast agent within the region of interest 170 may also be determined. The information from the temporal profile may then be used to normalize the calculated value of the gradient of the contrast agent along the one or more portions Pₐ - P_{d} of the lumen. The value of the calculated gradient of the contrast agent along the lumen has been observed to depend on the concentration of the contrast agent that is injected into the vasculature. Clinical facilities may follow different protocols and in which different concentrations of contrast agent are injected. Thus, by using the information from the temporal profile to normalize the calculated value of the gradient, confounding effects arising from different clinical facilities using different concentrations of contrast agent may be compensated-for. In this example, the method further includes determining a temporal profile of the intensity of the injected contrast agent within the region of interest 170; and normalizing the calculated value of the gradient of the contrast agent along the one or more portions Pₐ - P_{d} of the lumen, based on an intensity of the injected contrast agent in the temporal profile.

The temporal profile of the intensity of the injected contrast agent within the region of interest 170 may be determined by calculating e.g. its average, or peak value in each of multiple reconstructed image slices in a temporal sequence. By way of some examples, the intensity of the injected contrast agent in the temporal profile that is used to perform the normalization may be the peak value, or the mean value.

The inventors have also observed that measurements of the gradient 110 of a contrast agent along the lumen, can depend upon the position along the lumen that is used to trigger the generation of the CT data that is used to calculate the gradient. This hampers a comparison of gradients that are calculated at different clinical facilities, and which may have different protocols for triggering the generation of the CT data.

In one example a planar planning image 190 is generated from tissue data that is isolated from the spectral CT data. The tissue data represents the lumen and one or more tissue landmarks. In this example, the method described with reference to Fig. 1 includes:
receiving spectral CT data representing a planar image 180 including the lumen and one or more anatomical landmarks, the anatomical landmarks including one or more bone landmarks and one or more tissue landmarks;
analyzing the spectral CT data representing the planar image 180, and isolating from said data, tissue data representing the lumen and the one or more tissue landmarks; and
outputting a planar planning image 190 representing the tissue data.

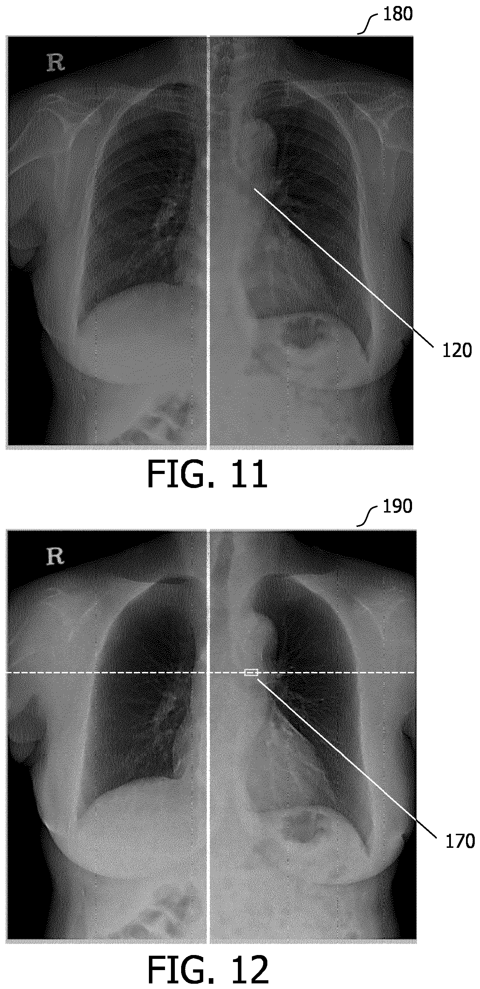

This example is described with reference to Fig. 11 and Fig. 12. Fig. 11 is an example of a planar image 180 including a lumen 120 and one or more anatomical landmarks, in accordance with some aspects of the present disclosure. As may be seen in the Fig. 11 image, both tissue landmarks, such as the trachea, and bone landmarks, such as the ribs, are visible in the planar image illustrated in Fig. 11. As may be appreciated, the bone landmarks such as the ribs, tend to confound the identification of tissue landmarks. Consequently, it can be difficult to plan the acquisition of CT data in order to determine a gradient of an injected contrast agent in a lumen using the planar image illustrated in Fig. 11, or likewise a using conventional CT image that also represents both tissue and bone landmarks. Fig. 12 is an example of a planar planning image 190 representing tissue data, in accordance with some aspects of the present disclosure. In the planar planning image illustrated in Fig. 12, the bone landmarks have been suppressed. Thus, the planar planning image illustrated in Fig. 12 may be used to more-easily identify a lumen of interest, and also to more easily identify a region of interest in the lumen of interest to use to trigger the generation of the spectral CT data 130₁, 130₂ representing the distribution of the injected contrast agent along the lumen 120.

In a related example, the method also includes:
identifying the at least one region of interest in the planar planning image 190; and
wherein the identifying the at least one region of interest is performed automatically, or in response to user input received from a user input device.

In this example, the region of interest may be identified by displaying a marker in the planar planning image, for example. As illustrated in Fig. 12, a marker such as the rectangle is located in the descending aorta at the level of the bifurcation of the trachea, which may serve as a region of interest for use in triggering the generation of the spectral CT data 130₁, 130₂ in a consistent manner. The bifurcation of the trachea serves as a reliable tissue landmark for use in triggering the generation of the spectral CT data 130₁, 130₂ across different subjects. Since in this example, the region of interest is identified in the planar planning image 190 representing tissue data, i.e. in an image in which the bone landmarks have been suppressed, the region of interest may be identified in a more consistent manner. The planar planning image 190 illustrated in Fig. 12 also includes a second marker in the form of a horizontal dashed line. The position of the second marker corresponds to the axial position of the image slice illustrated in Fig. 10, and also assists a physician to plan the procedure.

The region of interest 170 illustrated in Fig. 12 may be defined manually by means of a user operating a user input device in combination with the displayed planar planning image 190. The region of interest 170 illustrated in Fig. 12 may alternatively be defined automatically, for example by using a feature detector, or a trained neural network. The neural network may be trained to detect a region of interest such as the bifurcation of the trachea, for example.

In another example, a time-dependent value is calculated for the gradient 110 of the contrast agent along the lumen. In this example, the received spectral CT data 130₁, 130₂ represents a distribution of an injected contrast agent along the lumen at each of multiple points in time; and the method described with reference to Fig. 1 also includes:
analyzing S120 and the calculating S130, are performed for the data corresponding to each point in time to provide a time-dependent calculated value for the gradient 110 of the contrast agent along the one or more portions Pₐ - P_{d} of the lumen; and
wherein the method further comprises outputting the time-dependent calculated value for the gradient 110 of the contrast agent along the one or more portions Pₐ - P_{d} of the lumen.

The spectral CT data 130₁, 130₂ may be generated at each of multiple points in time by continuously acquiring data for a period of time after the contrast agent has been injected into the vasculature. The time-dependant spectral CT data may then be analyzed the same manner as described above in the operation S120. The analysis may be performed in the projection domain, or in the image domain, and in the same manner as described above in the operation S120. For example, a temporal sequence of volumetric images representing the contrast agent attenuation data 140 may be reconstructed, and the value of a gradient 110 of the contrast agent along the lumen may be calculated using the data for each of the images. A spectral X-ray projection imaging system that includes a detector with a plurality of detector elements 240_{1..k} arranged along an axis of rotation 250 of the X-ray detector may be used to acquire the spectral CT data 130₁, 130₂ in this example. The detector elements 240_{1..k} may for example be arranged parallel to, or at an acute angle with respect to the axis of rotation. An example of this arrangement is illustrated in Fig. 4. This arrangement allows the X-ray detector 230 to capture spectral CT data in order to determine a gradient of an injected contrasts agent in a lumen that extends in a direction along the axis of rotation, as is the case in some of the vessels in the heart, for instance.

Various advantages are associated with the provision of a time-dependent calculated value for the gradient 110 of the contrast agent along the lumen. For instance, the need to accurately trigger the generation of the spectral CT data 130₁, 130₂, is obviated. Moreover, the time-dependent calculated value for the gradient 110 may be processed in order to provide a more robust value for the gradient. For example, an average value for the gradient may be calculated over a period of time in order to eliminate spurious effects and to reduce the effects of noise.

In another example, a blood flow parameter may be determined for the lumen. The blood flow parameter may be the FFR, for example. In this example, the method described with reference to Fig. 1 also includes:
determining a value of a blood flow parameter for the lumen, using the calculated value of the gradient 110 of the contrast agent along the one or more portions Pₐ - P_{d} of the lumen 120.

In this example, the value of the blood flow parameter may be determined using a lookup table that provides a correspondence between the calculated value of the gradient 110 of the contrast agent along the lumen, and the value of the blood flow parameter. The blood flow parameter may for instance be the fractional flow reserve, FFR. The correspondence between the gradient and the blood flow parameter value may be determined empirically, or from a model representing the lumen. Lookup tables for blood flow parameters such as the FFR, the instantaneous wave-free ratio "iFR", the Coronary Flow Reserve "CFR", the Thrombolysis in Myocardial Infarction "TIMI" flow grade, the Index of Microvascular Resistance "IMR", and the Hyperemic Microvascular Resistance index "HMR" may also be determined using a lookup table in a similar manner.

In another example, a computer program product, is provided. The computer program product comprises instructions which when executed by one or more processors, cause the one or more processors to carry out a method of calculating a value of a contrast agent attenuation gradient 110 for a lumen 120 in a vasculature. The method comprises:
receiving S110 spectral computed tomography, CT, data 130₁, 130₂ representing a distribution of an injected contrast agent along the lumen 120, the spectral CT data defining X-ray attenuation at a plurality of energy intervals ΔE_{1..m};
analyzing S120 the spectral CT data to isolate from the spectral CT data, contrast agent attenuation data 140 representing the distribution of the contrast agent along the lumen 120;
calculating S130, from the contrast agent attenuation data 140, a value of a gradient 110 of the contrast agent along one or more portions Pₐ - P_{d} of the lumen 120, to provide the value of the contrast agent attenuation gradient 110.

In another example, a system 200 for calculating a value of a contrast agent attenuation gradient 110 for a lumen 120 in a vasculature, is provided. The system includes one or more processors 210 configured to:
receive S110 spectral computed tomography, CT, data 130₁, 130₂ representing a distribution of an injected contrast agent along the lumen 120, the spectral CT data defining X-ray attenuation at a plurality of energy intervals ΔE_{1..m};
analyze S120 the spectral CT data to isolate from the spectral CT data, contrast agent attenuation data 140 representing the distribution of the contrast agent along the lumen 120;
calculate S 130, from the contrast agent attenuation data 140, a value of a gradient 110 of the contrast agent along one or more portions Pₐ - P_{d} of the lumen 120, to provide the value of the contrast agent attenuation gradient 110.

An example of the system 200 is illustrated in Fig. 2. It is noted that the system 200 may also include one or more of: a spectral X-ray imaging system for generating the spectral CT data that is received in the operation S110, such as for example the spectral CT imaging system 220 illustrated in Fig. 2; a monitor 300 for displaying the calculated value of the gradient of the contrast agent along the lumen, reconstructed images, reconstructed image slices, and so forth; a patient bed 310; an injector (not illustrated in Fig. 2) for injecting a contrast agent into the vasculature; and a user input device configured to receive user input relating to the operations performed by the system, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of calculating a value of a contrast agent transluminal attenuation gradient (110) for a lumen (120) in a vasculature, the method comprising:
receiving (S110) spectral computed tomography, CT, data (130₁, 130₂) representing a distribution of an injected contrast agent along the lumen (120), the spectral CT data defining X-ray attenuation at a plurality of energy intervals (ΔE_{1..m});
analyzing (S120) the spectral CT data to isolate from the spectral CT data, contrast agent attenuation data (140) representing the distribution of the contrast agent along the lumen (120);
calculating, from the contrast agent attenuation data (140), an average value of the contrast agent attenuation across the lumen (120), at a plurality of positions (Pₐ, P_{1..4}, P_{d}) along the lumen; and
calculating (S130) a value of a gradient (110) of the contrast agent along one or more portions (Pₐ - P_{d}) of the lumen using the average values of the contrast agent attenuation across the lumen, to provide the value of the contrast agent transluminal attenuation gradient (110).

2. The computer-implemented method according to claim 1, further comprising identifying a centerline (150) of the lumen (120); and wherein the calculating (S130) an average value of the contrast agent attenuation across the lumen (120), at a plurality of positions (Pₐ, P_{1..4}, P_{d}) along the lumen; is performed at the plurality of positions (Pₐ, P_{1..4}, P_{d}) along the centerline of the lumen.

3. The computer-implemented method according to any one of claims 1 - 2, wherein the analyzing (S120) the spectral CT data, further comprises:
analyzing the spectral CT data (130₁, 130₂) to isolate from the spectral CT data, artifact attenuation data representing attenuation arising from one or more artifacts; and
weighting the isolated contrast agent attenuation data such that an impact of the artifacts on the isolated contrast agent attenuation data (140) is reduced.

4. The computer-implemented method according to claim 3, wherein the one or more spectral CT data energy intervals (ΔE_{1..m}) comprises a relatively lower energy interval (ΔE₁) and a relatively higher energy interval (ΔE₂); and
wherein the contrast agent attenuation data (140) is isolated from the spectral CT data (130₁, 130₂) by subtracting the spectral CT data corresponding to the relatively higher energy interval (ΔE₂) from the spectral CT data corresponding to the relatively lower energy interval (ΔE₁); and
wherein the artifact attenuation data (140) is provided by the spectral CT data corresponding to the relatively higher energy interval (ΔE₂).

5. The computer-implemented method according to any one of claims 1 - 4, wherein the contrast agent attenuation data comprises (140) projection data, or wherein the contrast agent attenuation data (140) comprises reconstructed image data; and
wherein the calculating (S130) a value of a gradient (110) of the contrast agent along one or more portions (Pₐ - P_{d}) of the lumen, is performed in the projection domain, or in the image domain, respectively.

6. The computer-implemented method according to any one of claims 1 - 5, wherein the method further comprises:
receiving spectral CT data representing a flow of the injected contrast agent in the lumen (120);
analyzing the spectral CT data representing the flow of the injected contrast agent in the lumen to isolate from said data, contrast agent attenuation flow data (160) representing the flow of the injected contrast agent in the lumen (120); and
wherein the method further comprises:
triggering a generation of the spectral CT data (130₁, 130₂) representing the distribution of the injected contrast agent along the lumen (120), if the injected contrast agent in the lumen represented in the contrast agent attenuation flow data satisfies a predetermined threshold condition.

7. The computer-implemented method according to claim 6, wherein the triggering a generation of the spectral CT data (130₁, 130₂) representing the distribution of the injected contrast agent along the lumen (120), comprises initiating the generation of the spectral CT data (130₁, 130₂) representing the distribution of the injected contrast agent along the lumen (120), if an intensity of the injected contrast agent represented in the contrast agent attenuation flow data (160) exceeds a predetermined threshold value at a predetermined position in the lumen (120).

8. The computer-implemented method according to claim 7, wherein the received spectral CT data representing the flow of the injected contrast agent in the lumen (120) comprises projection data that is generated during a rotation of an X-ray source detector arrangement (270, 230) around the lumen (120), and wherein the method further comprises:
reconstructing the projection data into an image slice; and wherein the initiating the generation of the spectral CT data (130₁, 130₂), comprises analyzing an image intensity within a region of interest (170) in the reconstructed image slice, and initiating the generation of the spectral CT data (130₁, 130₂) representing the distribution of the injected contrast agent along the lumen (120), if the image intensity within the region of interest exceeds the predetermined threshold value.

9. The computer-implemented method according to any one of claims 7-8, further comprising determining a temporal profile of the intensity of the injected contrast agent within the region of interest (170); and normalizing the calculated value of the gradient of the contrast agent along the one or more portions (Pₐ - P_{d}) of the lumen, based on an intensity of the injected contrast agent in the temporal profile.

10. The computer-implemented method according to any previous claim, wherein the method further comprises:
receiving spectral CT data representing a planar image (180) including the lumen (120) and one or more anatomical landmarks, the anatomical landmarks including one or more bone landmarks and one or more tissue landmarks;
analyzing the spectral CT data representing the planar image (180), and isolating from said data, tissue data representing the lumen and the one or more tissue landmarks; and
outputting a planar planning image (190) representing the tissue data.

11. The computer-implemented method according to claim 10 when dependent on claim 8 or claim 9, wherein the method further comprises:
identifying the at least one region of interest in the planar planning image (190); and
wherein the identifying the at least one region of interest is performed automatically, or in response to user input received from a user input device.

12. The computer-implemented method according to any previous claim, wherein the method further comprises: determining a value of a blood flow parameter for the lumen, using the calculated value of the gradient (110) of the contrast agent along the one or more portions (Pₐ - P_{d}) of the lumen (120).

## Patentansprüche

1. Computerimplementiertes Verfahren zum Berechnen eines Wertes eines transluminalen Dämpfungsgradienten (110) eines Kontrastmittels für ein Lumen (120) in einem Gefäßsystem, wobei das Verfahren Folgendes umfasst:
Empfangen (S110) von spektralen Computertomographie-, CT-, Daten (130₁, 130₂), die eine Verteilung eines injizierten Kontrastmittels entlang des Lumens (120) darstellen, wobei die spektralen CT-Daten die Röntgenendämpfung in einer Vielzahl von Energieintervallen (ΔE_{1..m}) definieren;
Analysieren (S120) der spektralen CT-Daten, um aus den spektralen CT-Daten Kontrastmittel-Dämpfungsdaten (140) zu isolieren, die die Verteilung des Kontrastmittels entlang des Lumens (120) darstellen;
Berechnen eines Mittelwerts der Kontrastmitteldämpfung über das Lumen (120) an einer Vielzahl von Positionen (Pₐ, P_{1..4}, P_{d}) entlang des Lumens aus den Kontrastmittel-Dämpfungsdaten (140); und
Berechnen (S130) eines Wertes eines Gradienten (110) des Kontrastmittels entlang eines oder mehrerer Abschnitte (Pₐ - P_{d}) des Lumens unter Verwendung der Mittelwerte der Kontrastmitteldämpfung über das Lumen, um den Wert des transluminalen Kontrastmittel-Dämpfungsgradienten (110) zu erhalten.

2. Computerimplementiertes Verfahren nach Anspruch 1, das ferner Identifizieren einer Mittellinie (150) des Lumens (120) umfasst; und wobei das Berechnen (S130) eines Mittelwerts der Kontrastmitteldämpfung über das Lumen (120) an einer Vielzahl von Positionen (Pₐ, P_{1..4}, P_{d}) entlang des Lumens an der Vielzahl von Positionen (Pₐ, P_{1..4}, P_{d}) entlang der Mittellinie des Lumens durchgeführt wird.

3. Computerimplementiertes Verfahren nach einem der Ansprüche 1-2, wobei das Analysieren (S120) der spektralen CT-Daten ferner Folgendes umfasst:
Analysieren der spektralen CT-Daten (130₁, 130₂), um aus den spektralen CT-Daten Artefaktdämpfungsdaten zu isolieren, die die Dämpfung aufgrund eines oder mehrerer Artefakte darstellen; und
Gewichten der isolierten Kontrastmittel-Dämpfungsdaten, um einen Einfluss von Artefakten auf die isolierten Kontrastmittel-Dämpfungsdaten (140) zu reduzieren.

4. Computerimplementiertes Verfahren nach Anspruch 3, wobei das eine oder die mehreren spektralen CT-Datenenergieintervalle (ΔE_{1..m}) ein relativ niedrigeres Energieintervall (ΔE₁) und ein relativ höheres Energieintervall (ΔE₂) umfassen; und
wobei die Kontrastmittel-Dämpfungsdaten (140) aus den spektralen CT-Daten (130₁, 130₂) isoliert werden, indem die spektralen CT-Daten, die dem relativ höheren Energieintervall (ΔE₂) entsprechen, von den spektralen CT-Daten, die dem relativ niedrigeren Energieintervall (ΔE₁ ) entsprechen, subtrahiert werden; und
wobei die Artefaktdämpfungsdaten (140) durch die spektralen CT-Daten, die dem relativ höheren Energieintervall (ΔE₂) entsprechen, bereitgestellt werden.

5. Computerimplementiertes Verfahren nach einem der Ansprüche 1-4, wobei die Kontrastmitteldämpfungsdaten (140) Projektionsdaten umfassen oder wobei die Kontrastmittel-Dämpfungsdaten (140) rekonstruierte Bilddaten umfassen; und
wobei das Berechnen (S130) eines Wertes eines Gradienten (110) des Kontrastmittels entlang eines oder mehrerer Abschnitte (Pₐ - P_{d}) des Lumens im Projektionsbereich bzw. im Bildbereich durchgeführt wird.

6. Computerimplementiertes Verfahren nach einem der Ansprüche 1-5, wobei das Verfahren ferner Folgendes umfasst:
Empfangen von spektralen CT-Daten, die einen Fluss des injizierten Kontrastmittels im Lumen (120) darstellen;
Analysieren der spektralen CT-Daten, die den Fluss des injizierten Kontrastmittels im Lumen darstellen, um aus diesen Daten Kontrastmittel-Dämpfungsflussdaten (160) zu isolieren, die den Fluss des injizierten Kontrastmittels im Lumen (120) darstellen; und
wobei das Verfahren ferner umfasst:
Auslösen einer Generierung der spektralen CT-Daten (130₁, 130₂), die die Verteilung des injizierten Kontrastmittels entlang des Lumens (120) darstellen, wenn das injizierte Kontrastmittel im Lumen, dargestellt in den Kontrastmittel-Dämpfungsflussdaten, eine vorbestimmte Schwellenbedingung erfüllt.

7. Computerimplementiertes Verfahren nach Anspruch 6, wobei das Auslösen einer Generierung der spektralen CT-Daten (130₁, 130₂), die die Verteilung des injizierten Kontrastmittels entlang des Lumens (120) darstellen, Initiieren der Generierung der spektralen CT-Daten (130₁, 130₂) umfasst, die die Verteilung des injizierten Kontrastmittels entlang des Lumens (120) darstellen, wenn eine Intensität des injizierten Kontrastmittels, die in den Kontrastmittel-Dämpfungsflussdaten (160) dargestellt ist, an einer vorbestimmten Position im Lumen (120) einen vorbestimmten Schwellenwert überschreitet.

8. Computerimplementiertes Verfahren nach Anspruch 7, wobei die empfangenen spektralen CT-Daten, die den Fluss des injizierten Kontrastmittels im Lumen (120) darstellen, Projektionsdaten umfassen, die während einer Rotation einer Röntgenquellen-Detektor-Anordnung (270, 230) um das Lumen (120) generiert werden, und wobei das Verfahren ferner Folgendes umfasst:
Rekonstruieren der Projektionsdaten zu einem Bildschnitt; und wobei das Einleiten der Generierung der spektralen CT-Daten (130₁, 130₂) Analysieren einer Bildintensität innerhalb eines Bereichs von Interesse (170) im rekonstruierten Bildschnitt und Einleiten der Generierung der spektralen CT-Daten (130₁, 130₂), die die Verteilung des injizierten Kontrastmittels entlang des Lumens (120) darstellen, wenn die Bildintensität innerhalb des Bereichs von Interesse den vorgegebenen Schwellenwert überschreitet, umfasst.

9. Computerimplementiertes Verfahren nach einem der Ansprüche 7-8, ferner umfassend das Bestimmen eines zeitlichen Profils der Intensität des injizierten Kontrastmittels innerhalb des Bereichs von Interesse (170); und das Normalisieren des berechneten Wertes des Gradienten des Kontrastmittels entlang des einen oder mehrerer Abschnitte (Pₐ - P_{d}) des Lumens auf der Grundlage einer Intensität des injizierten Kontrastmittels im zeitlichen Profil.

10. Computerimplementiertes Verfahren nach einem vorstehenden Anspruch, wobei das Verfahren weiter Folgendes umfasst:
Empfangen von spektralen CT-Daten, die ein planares Bild (180) einschließlich des Lumens (120) und eines oder mehrerer anatomischer Orientierungspunkte darstellen, wobei die anatomischen Orientierungspunkte einen oder mehrere Knochenorientierungspunkte und einen oder mehrere Gewebeorientierungspunkte einschließen;
Analysieren der spektralen CT-Daten, die das planare Bild (180) darstellen, und Isolieren von Gewebedaten, die das Lumen und den einen oder die mehreren Gewebeorientierungspunkte darstellen, aus diesen Daten; und
Ausgeben eines planaren Planungsbildes (190), das die Gewebedaten darstellt.

11. Computerimplementiertes Verfahren nach Anspruch 10, wenn von Anspruch 8 oder Anspruch 9 abhängig, wobei das Verfahren ferner Folgendes umfasst:
Identifizieren des mindestens einen relevanten Bereichs von Interesse im planaren Planungsbild (190); und
wobei das Identifizieren des mindestens einen relevanten Bereichs von Interesse automatisch oder als Reaktion auf Benutzereingaben durchgeführt wird, die von einer Benutzereingabevorrichtung empfangen werden.

12. Computerimplementiertes Verfahren nach einem vorstehenden Anspruch, wobei das Verfahren ferner Folgendes umfasst: Bestimmen eines Wertes eines Blutflussparameters für das Lumen unter Verwendung des berechneten Wertes des Gradienten (110) des Kontrastmittels entlang des einen oder der mehreren Abschnitte (Pₐ - P_{d}) des Lumens (120).

## Revendications

1. Procédé mis en œuvre par ordinateur de calcul d'une valeur d'un gradient d'atténuation transluminale d'agent de contraste (110) pour une lumière (120) dans un système vasculaire, le procédé comprenant :
la réception (S110) de données de tomodensitométrie (CT) spectrale (130₁, 130₂) représentant une distribution d'un agent de contraste injecté le long de la lumière (120), les données de CT spectrale définissant une atténuation des rayons X à une pluralité d'intervalles d'énergie (ΔE_{1..m}) ;
l'analyse (S120) des données de CT spectrale pour isoler à partir des données de CT spectrale, des données d'atténuation d'agent de contraste (140) représentant la distribution de l'agent de contraste le long de la lumière (120) ;
le calcul, à partir des données d'atténuation d'agent de contraste (140), d'une valeur moyenne de l'atténuation d'agent de contraste à travers la lumière (120), à une pluralité de positions (Pₐ, P_{1..4}, P_{d}) le long de la lumière ; et
le calcul (S130) d'une valeur d'un gradient (110) de l'agent de contraste le long d'une ou plusieurs parties (Pₐ - P_{d}) de la lumière à l'aide des valeurs moyennes de l'atténuation d'agent de contraste à travers la lumière, pour fournir la valeur du gradient d'atténuation transluminale d'agent de contraste (110).

2. Procédé mis en œuvre par ordinateur selon la revendication 1, comprenant en outre l'identification d'une ligne centrale (150) de la lumière (120) ; et dans lequel le calcul (S130) d'une valeur moyenne de l'atténuation d'agent de contraste à travers la lumière (120), à une pluralité de positions (Pₐ, P_{1..4}, P_{d}) le long de la lumière est effectué à la pluralité de positions (Pₐ, P_{1..4}, P_{d}) le long de la ligne centrale de la lumière.

3. Procédé mis en œuvre par ordinateur selon l'une quelconque des revendications 1-2, dans lequel l'analyse (S120) des données de CT spectrale comprend en outre :
l'analyse des données de CT spectrale (130₁, 130₂) pour isoler, des données de CT spectrale, des données d'atténuation d'artefacts représentant l'atténuation découlant d'un ou plusieurs artefacts ; et
la pondération des données d'atténuation d'agent de contraste isolées de manière à réduire un impact des artefacts sur les données d'atténuation d'agent de contraste (140) isolées.

4. Procédé mis en œuvre par ordinateur selon la revendication 3, dans lequel les un ou plusieurs intervalles d'énergie de données de CT spectrale (ΔE_{1..m}) comprennent un intervalle d'énergie relativement inférieure (ΔE₁) et un intervalle d'énergie relativement supérieure (ΔE₂) ; et
dans lequel les données d'atténuation d'agent de contraste (140) sont isolées des données de CT spectrale (130₁, 130₂) en soustrayant les données de CT spectrale correspondant à l'intervalle d'énergie relativement supérieure (ΔE₂) des données de CT spectrale correspondant à l'intervalle d'énergie relativement inférieure (ΔE₁) ; et
dans lequel les données d'atténuation d'artefacts (140) sont fournies par les données de CT spectrale correspondant à l'intervalle d'énergie relativement supérieure (ΔE₂).

5. Procédé mis en œuvre par ordinateur selon l'une quelconque des revendications 1-4, dans lequel les données d'atténuation d'agent de contraste comprennent (140) des données de projection, ou dans lequel les données d'atténuation d'agent de contraste (140) comprennent des données d'image reconstruites ; et
dans lequel le calcul (S130) d'une valeur d'un gradient (110) de l'agent de contraste le long d'une ou plusieurs parties (Pₐ - P_{d}) de la lumière, est effectué respectivement dans le domaine de projection ou dans le domaine d'image.

6. Procédé mis en œuvre par ordinateur selon l'une quelconque des revendications 1 à 5, dans lequel le procédé comprend en outre :
la réception de données de CT spectrale représentant un flux de l'agent de contraste injecté dans la lumière (120) ;
l'analyse des données de CT spectrale représentant le flux de l'agent de contraste injecté dans la lumière pour isoler, desdites données, des données de flux d'atténuation d'agent de contraste (160) représentant le flux de l'agent de contraste injecté dans la lumière (120) ; et
dans lequel le procédé comprend en outre :
le déclenchement d'une génération des données de CT spectrale (130₁, 130₂) représentant la distribution de l'agent de contraste injecté le long de la lumière (120), si l'agent de contraste injecté dans la lumière représenté dans les données de flux d'atténuation d'agent de contraste satisfait à une condition de seuil prédéterminée.

7. Procédé mis en œuvre par ordinateur selon la revendication 6, dans lequel le déclenchement d'une génération des données de CT spectrale (130₁, 130₂) représentant la distribution de l'agent de contraste injecté le long de la lumière (120) comprend l'initiation de la génération des données de CT spectrale (130₁, 130₂) représentant la distribution de l'agent de contraste injecté le long de la lumière (120), si une intensité de l'agent de contraste injecté représentée dans les données de flux d'atténuation d'agent de contraste (160) dépasse une valeur seuil prédéterminée à une position prédéterminée dans la lumière (120).

8. Procédé mis en œuvre par ordinateur selon la revendication 7, dans lequel les données de CT spectrale reçues représentant le flux de l'agent de contraste injecté dans la lumière (120) comprennent des données de projection générées lors d'une rotation d'un agencement détecteur de source de rayons X (270, 230) autour de la lumière (120), et dans lequel le procédé comprend en outre :
la reconstruction des données de projection dans une tranche d'image ; et dans lequel l'initiation de la génération des données de CT spectrale (130₁, 130₂) comprend l'analyse d'une intensité d'image dans une région d'intérêt (170) dans la tranche d'image reconstruite, et l'initiation de la génération des données de CT spectrale (130₁, 130₂) représentant la distribution de l'agent de contraste injecté le long de la lumière (120), si l'intensité d'image dans la région d'intérêt dépasse la valeur de seuil prédéterminée.

9. Procédé mis en œuvre par ordinateur selon l'une quelconque des revendications 7-8, comprenant en outre la détermination d'un profil temporel de l'intensité de l'agent de contraste injecté dans la région d'intérêt (170) ; et la normalisation de la valeur calculée du gradient de l'agent de contraste le long des une ou plusieurs parties (Pₐ - P_{d}) de la lumière, sur la base d'une intensité de l'agent de contraste injecté dans le profil temporel.

10. Procédé mis en œuvre par ordinateur selon une quelconque revendication précédente, dans lequel le procédé comprend en outre :
la réception de données de CT spectrale représentant une image planaire (180) incluant la lumière (120) et un ou plusieurs repères anatomiques, les repères anatomiques incluant un ou plusieurs repères osseux et un ou plusieurs repères tissulaires ;
l'analyse des données de CT spectrale représentant l'image planaire (180) et l'isolation, desdites données, de données tissulaires représentant la lumière et un ou plusieurs repères tissulaires ; et
la génération d'une image de planification planaire (190) représentant les données tissulaires.

11. Procédé mis en œuvre par ordinateur selon la revendication 10 lorsqu'elle dépend de la revendication 8 ou de la revendication 9, dans lequel le procédé comprend en outre :
l'identification de l'au moins une région d'intérêt dans l'image de planification planaire (190) ; et
dans lequel l'identification de l'au moins une région d'intérêt est effectuée automatiquement ou en réponse à une entrée utilisateur reçue d'un dispositif d'entrée utilisateur.

12. Procédé mis en œuvre par ordinateur selon une quelconque revendication précédente, dans lequel le procédé comprend en outre : la détermination d'une valeur d'un paramètre de flux sanguin pour la lumière, à l'aide de la valeur calculée du gradient (110) de l'agent de contraste le long des une ou plusieurs parties (Pₐ - P_{d}) de la lumière (120).
